# EUROPEAN PATENT APPLICATION

(11) **EP 4 640 193 A1**
(43) Date of publication of application: **29.10.2025**
(21) Application number: 24172093.7
(22) Date of filing: 24.04.2024
(51) Int. Cl.: A61F 7/00, A61F 7/02, A61F 7/10, A61F 7/08

(54) **COLD AND HOT COMPRESS DEVICE WITH DUAL WATER CIRCUIT DESIGN**

(71) Applicant: Hwang, Chin-Hwa, Taipei City (TW)
(72) Inventor: Hwang, Chin-Hwa, Taipei City (TW)
(74) Representative: Zeitler Volpert Kandlbinder Patentanwälte Partnerschaft mbB

(57) **Abstract**

A hot and cold compress device with a dual water circuit design includes a main system, a hot and cold water compress pad and an electric heating compress pad, the two compress pads interchangeably is connected to the main system and attachably fixed to the surface of an affected part of a human body. The hot and cold water compress pad contains two separate water channels. Cold water and/or hot water can be obtained from the main system before executing a quick cold compress, a hot and cold compress switch or a hot compress, and the electric heating compress pad can obtain electric power from the main system to execute a quick hot compress.

## Description

### TECHNICAL FIELD OF THE DISCLOSURE

The present disclosure relates to a hot and cold compress device, which includes a main system, a hot and cold water compress pad, and an electric heating compress pad. The main system has a cold water chamber and a hot and cold water chamber, the hot and cold water compress pad has a cold water channel and a hot and cold water channel independent of each other, and the hot and cold water compress pad and the electric heating compress pad are interchangeably connected to the main system in order to achieve the function of performing a quick hot compress, a quick cold compress, or a quick switch between the hot and cold compresses.

### DESCRIPTION OF RELATED ART

A hot and cold compress device is a device using refrigerating and heating components as the temperature generating source together with cold water or hot water as the cold and hot conduction medium. The cold water and the hot water can flow in a pre-set pipeline to a compress pad which is attached to human body to provide the cold compress or hot compress effect to human body.

In relevant prior art structures as disclosed in U.S. Pat. Nos. 3,967,627 and 5,344,436, the structure includes a water tank and an applicator pad which are communicated to each other through a set of pipes, and the water tank is equipped with heating and refrigerating components, and a peristaltic pump is used to pump the cold water or hot water contained in the water tank to the applicator pad through the set of pipes, and then circulate the cold or hot water into the water tank, so that users can place the applicator pad against their skin to obtain the cold compress or hot compress effect.

In the above related art, since the cold compress or hot compress function is accomplished through the same circulation pipeline and water tank, therefore the temperature adjustment on the applicator pad is very slow and inaccurate, especially when it is necessary to switch the function of the applicator pad from cold compress to hot compress, or from hot compress to cold compress, users have to wait for a long time, and thus the efficiency is poor.

As disclosed in U.S. Pat. Application No. 20200008975A1, the water tank is separated into a cold water storage device and a hot water storage device, and different pumps are used to drive cold water or hot water to flow in the applicator pad in order to overcome the above problem of poor efficiency when switching between the cold compress and the hot compress. However, the flow channel for circulating cold water or hot water is still the same flow channel of the applicator pad of this prior art. In other words, when a user needs to switch from cold compress to hot compress or from hot compress to cold compress, the user still has to wait for a long time.

As disclosed in U.S. Pat. Application No. 20040068309A1, a mixing valve is used to mix hot fluid and cold fluid to a selected temperature, and then the mixed fluid is transferred to a therapy pad, so the therapy pad can be adjusted to the desired temperature in a relatively short period of time. However, the therapy pad also uses the same group of channels to circulate the fluids, therefore after the hot and cold fluids of different temperatures are mixed, the mixed fluid is circulated back to a container, whether it flows to the cold water container or hot water container, it will cause a big change in the original temperature of the cold water or hot water, resulting in energy loss and slow and inaccurate temperature adjustment in the subsequent mixing processes.

In U.S. Pat. Application No. 20180369015A1, another method of switching the temperature of the cold compress and hot compress is disclosed, and its technological content mainly consists of setting up an electric heating plate and a cold water channel on the therapy pad. When the hot compress is applied, the flow of cold water is stopped and the electric heating plate is activated. This arrangement enables a quick switch between cold -compress and hot compress on one hand, and enables the cold water storage device of the cooling system to maintain a low temperature on the other hand. However, when the electric heating plate is activated, there is cold water trapped in the therapy pad, causing the drawback that the electric heating plate has to gradually heat the cold water trapped in the therapy pad to the selected temperature before it can slowly achieve the effect of hot compress. This related art fails to provide a quick hot compress function.

In view of the aforementioned drawbacks, mainly the inefficient hot and cold compress switch function, the present discloser aims at three major functions of a quick cold compress, a quick switch between hot compress and cold compress and a quick hot compress, and improves the main system and the compress pad to allow users to set more flexible and accurate presentation of the set the temperature of the compress pad more flexibly and accurately, and thus improve the comfort of use.

### SUMMARY OF THE DISCLOSURE

The main objective of the present disclosure is to provide a hot and cold compress device with a dual water circuit design, the hot and cold compress device includes a main system separated into a cold water chamber and a hot and cold water chamber, and at least two compress pads interchangeably connected to the main system, and the two compress pads are flexible. The cold water chamber of the main system can be refrigerated quickly, and the hot and cold water chamber can be refrigerated or heated quickly. The two compress pads include a hot and cold water compress pad and an electric heating compress pad, and the hot and cold water compress pad contains a cold water channel and a hot and cold water channel independent of each other. By the two compress pads interchangeably installed to the main system, the function of a quick cold compress, a hot and cold compress switch or a quick hot compress can be accomplished.

To achieve the aforementioned objective, the present disclosure provides a hot and cold compress device with a dual water circuit design, which includes a main system and at least two compress pads, the two compress pads are flexible and attachably fixed to a surface of an affected part of human body, one of the compress pads is a hot and cold water compress pad, and the other compress pad is an electric heating compress pad.
The main system includes a casing, which is separated into a cold water chamber provided for storing cold water and installed with a refrigeration module, a hot and cold water chamber for storing cold water or hot water and installed with a heating and refrigerating module, an accommodation space formed between the cold water chamber and the hot and cold water chamber and provided for installing an electronic control system, a plurality of tube connectors disposed outside the accommodation space of the casing, and at least one USB port electrically connected to the electronic control system.
The electronic control system includes an operation interface installed outside the casing, and a motherboard, a cold water pump, a hot and cold water pump and a set of solenoid valves are installed in the accommodation space and electrically connected to the operation interface, and the refrigeration module and heating and the refrigerating module are electrically connected to the motherboard. The cold water pump is communicated to the set of solenoid valves and cold water chamber through a set of internal pipings, and the hot and cold water pump is communicated to the set of solenoid valves and hot and cold water chamber through a set of internal pipings, such that the cold water pump and the hot and cold water pump can pump water in the cold water chamber and the hot and cold water chamber to the tube connector.
The hot and cold water compress pad includes a hot and cold compress body substantially in the shape of a sheet, the hot and cold compress body includes a uniform temperature compress surface attachable to an affected part of a human body, and a backside opposite to the uniform temperature compress surface, a cold water channel and a hot and cold water channel separated from and independent of each other are disposed between the uniform temperature compress surface and the backside, the cold water channel and the hot and cold water channel are arranged in a winding shape, and each of the cold water channel and the hot and cold water channel is plugably/unplugably inserted to the tube connectors of the main system through a set of cold water tubes and a set of hot and cold water tubes respectively.
The electric heating compress pad includes a hot compress body substantially in the shape of a sheet, the hot compress body includes a uniform temperature compress surface attachable to an affected part of a human body, a backside opposite to the uniform temperature compress surface, an electric heating plate installed between the uniform temperature compress surface and the backside, and a flexible extension pad installed at an edge of the electric heating compress pad; wherein the tail end of the flexible extension pad is provided with a plug end and a USB transmission cable opposite to the tube connector and the USB port respectively and electrically coupled to the electric heating plate, such that the electric heating compress pad is plugably/unplugably inserted and positioned to the tube connector of the main system, and electrically coupled to the electronic control system of the main system after being inserted to the USB port through the USB transmission cable.
During a quick cold compress, the cold water tube and the hot and cold water tube of the hot and cold water compress pad are connected to the tube connector of the main system, and the operation interface is set, such that the motherboard drives the refrigeration module and the heating and refrigerating module while cooling the water stored in the cold water chamber and the hot and cold water chamber simultaneously, and the cold water pump, the hot and cold water pump and the solenoid valve are turned on or off to pump the cold water in the cold water chamber and the hot and cold water chamber through the tube connector, the cold water tube and the hot and cold water tube into the hot and cold compress body for circulation, and a quick cold compress is performed through the uniform temperature compress surface.
During a hot and cold compress switch, the cold water tube and the hot and cold water tube of the hot and cold compress body are coupled to the tube connector of the main system, and the operation interface is set, such that the motherboard can selectively drive the heating and refrigerating module to heat the water stored in the hot and cold water chamber, and/or drive the refrigeration module to cool the water stored in the cold water chamber, and the hot and cold water pump, the solenoid valve and/or the cold water pump are turned on or off to send the hot water and/or the cold water to the hot and cold water channel and/or the cold water channel of the hot and cold water compress pad for circulation, such that the hot and cold compress body can perform a hot and cold compress switch.
During a quick hot compress, the hot and cold water compress pad is removed from the main system, and the flexible extension pad of the electric heating compress pad is inserted to the tube connector and the USB port of the main system through the plug end and the USB transmission cable respectively, and the operation interface is set, such that the motherboard can transmit electric power to the electric heating plate through the USB port and the USB transmission cable for heating, to perform a quick hot compress at the uniform temperature compress surface of the hot compress body.

The technical characteristics of this disclosure will become apparent with the detailed description of preferred embodiments accompanied with the illustration of related drawings as follows.

### BRIEF DESCRIPTION OF THE DRAWINGS

FIG. 1 is a perspective view of the present disclosure;
FIG. 2 is an exploded view of a main system of the present disclosure;
FIG. 3 is a schematic view showing the internal structure of a main system of the present disclosure;
FIG. 4 is an exploded view of a hot and cold water compress pad of the present disclosure;
FIG. 5 is an exploded view of an electric heating compress pad of the present disclosure;
FIG. 6 is a schematic view showing the operation of a quick cold compress of the present disclosure;
FIG. 7 is a schematic view showing the operation of a hot and cold compress switch of the present disclosure;
FIG. 8 is a schematic view showing the operation of emptying cold water for a hot compress of the present disclosure;
FIG. 9 is a schematic view showing the operation of emptying hot water for a cold compress of the present disclosure;
FIG. 10 is a schematic view showing the installation of an electric heating plate in a hot and cold compress body of the present disclosure;
FIG. 11 is a schematic view showing the operation of a quick hot compress of the present disclosure;
FIG. 12 is a schematic view showing the installation of a fastener to the back of a hot and cold compress body and the back of a hot compress body respectively in accordance with the present disclosure; and
FIG. 13 is an exploded view of a cold water chamber and a hot and cold water chamber of the present disclosure.

### DETAILED DESCRIPTION OF THE DISCLOSURE

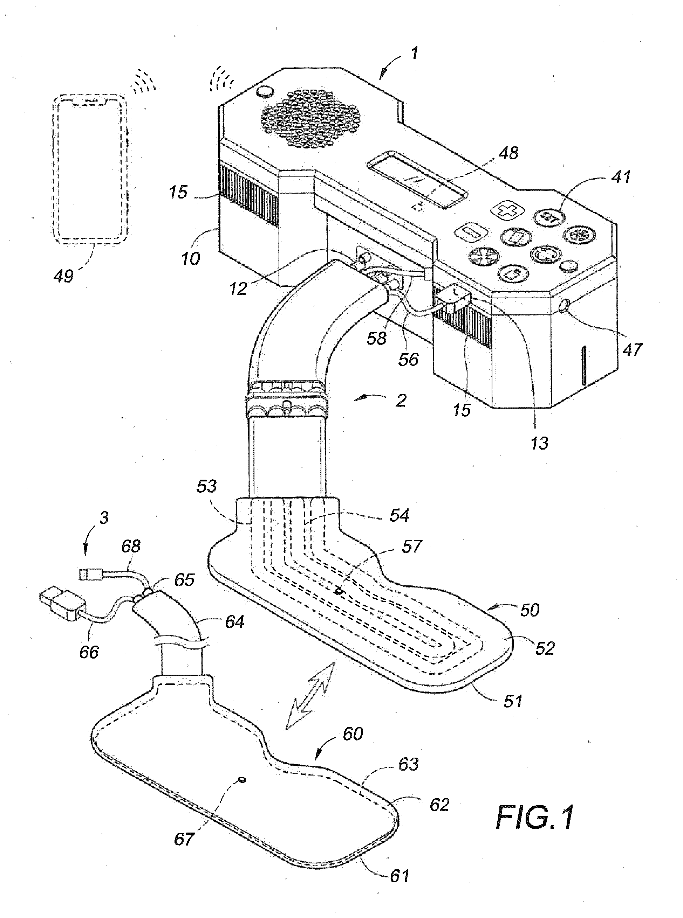

With reference to FIGS. 1 to 3 for a hot and cold compress device with a dual water circuit design in accordance with the present disclosure, the hot and cold compress device includes a main system 1 and at least two compress pads. The two compress pads are flexible and attachably fixed to the surface of an affected part of a human body, one of the two compress pads is a hot and cold water compress pad 2, the other one is an electric heating compress pad 3, and the hot and cold water compress pad 2 and the electric heating compress pad 3 are flatly fixed on the surface of the affected part of a human body, and interchangeably connected to the main system 1 to accomplish the function of a quick cold compress, a hot and cold compress switch or a quick hot compress with high efficiency.

In the figures, the main system 1 includes a casing 10 which is separated into a cold water chamber 20 and a hot and cold water chamber 30, and installed with an electronic control system 40. In the figures, the casing 10 of the main system 1 is substantially in the shape of a cuboid, the cold water chamber 20 and hot and cold water chamber 30 are disposed at two ends of the casing 10 respectively, and an accommodation space 11 is formed between the cold water chamber 20 and the hot and cold water chamber 30 of the casing 10 for accommodating the electronic control system 40.

The cold water chamber 20 includes a set of internal pipings 21, and a refrigeration module 22, the refrigeration module 22 is electrically connected to the electronic control system 40 and capable of refrigerating the cold water or room-temperature water stored in the cold water chamber 20.

The hot and cold water chamber 30 includes a set of internal pipings 31 and a heating and refrigerating module 32, the heating and refrigerating module 32 is electrically connected to the electronic control system 40 and capable of refrigerating or heating the water stored in the hot and cold water chamber 30.

In the figures, the heating and refrigerating module 32 is switched by using reverse current for heating or formed by a thermoelectric cooler (TEC). In some embodiments, the heating and refrigerating module 32 is formed by a refrigerating chip and an electric heating plate (not shown in the figure), such that the heating and refrigerating module 32 can selectively heat or refrigerate the water stored in the hot and cold water chamber 30.

The electronic control system 40 includes an operation interface 41 installed outside the casing 10, and a motherboard 42, a cold water pump 43, a hot and cold water pump 44, a set of solenoid valves 45 which are installed in the accommodation space 11. The operation interface 41, cold water pump 43, hot and cold water pump 44, and solenoid valve 45, as well as the aforementioned refrigeration module 22 and heating and refrigerating module 32 are electrically connected to the motherboard 42.

The cold water pump 43 is communicated to the set of solenoid valves 45 and cold water chamber 20 through the corresponding internal piping 21, the hot and cold water pump 44 is communicated to the set of solenoid valves 45 and the hot and cold water chamber 30 through the corresponding internal piping 31. In addition, the casing 10 of the main system 1 includes a plurality of tube connectors 12 installed outside the accommodation space 11 and connected to the cold water pump 43 and the hot and cold water pump 44 respectively, and at least one USB port 13 electrically connected to the motherboard 42.

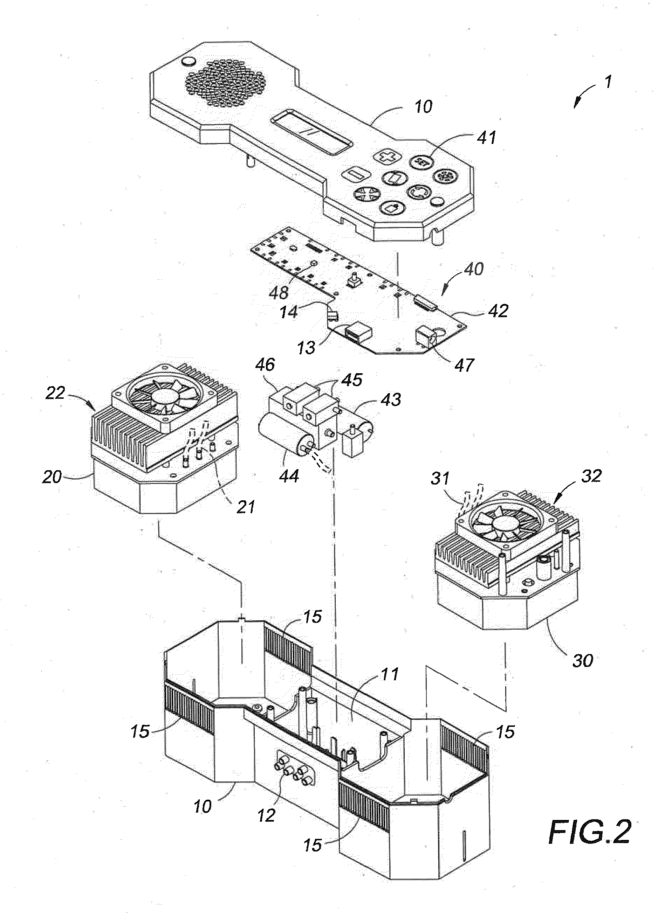

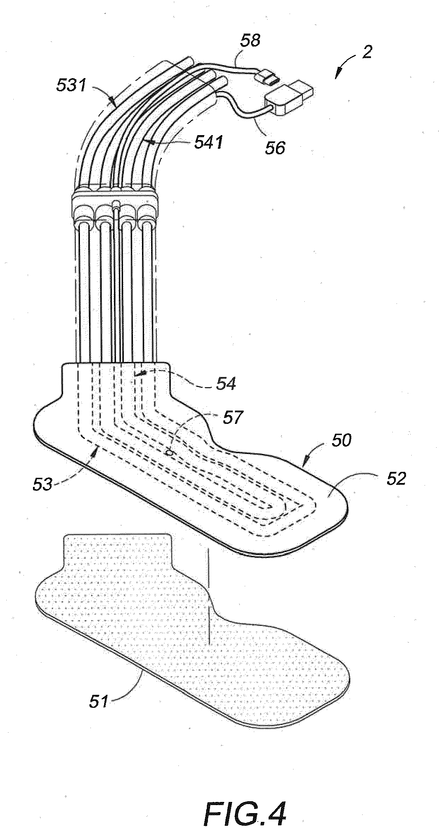

In FIGS. 2 and 4, the hot and cold water compress pad 2 includes a hot and cold compress body 50 substantially in the shape of a sheet, the hot and cold compress body 50 includes a uniform temperature compress surface 51 flatly attachable to an affected part of a human body, a backside 52 opposite to the uniform temperature compress surface 51, and a cold water channel 53 and a hot and cold water channel 54 separated from and independent of each other and disposed between the uniform temperature compress surface 51 and the backside 52, and the cold water channel 53 and the hot and cold water channel 54 are arranged in a winding shape inside the hot and cold compress body 50.

The cold water channel 53 is plugably/unplugably connected to the tube connector 12 of the main system 1 through a set of cold water tubes 531, and then communicated to the cold water pump 43 and the solenoid valve 45. When a user turns on the cold water pump 43 and the solenoid valve 45 through the operation interface 41 and motherboard 42, the cold water pump 43 will pump the water stored in the cold water chamber 20 to the cold water channel 53 inside the hot and cold compress body 50 through the set of tube connectors 12 and cold water tubes 531 and then circulate the water back into the cold water chamber 20.

The hot and cold water channel 54 is plugably/unplugably connected to the tube connector 12 of the main system 1 through a set of hot and cold water tubes 541 and then communicated to the hot and cold water pump 44 and the solenoid valve 45. After the hot and cold water pump 44 and the solenoid valve 45 are turned on, the hot and cold water pump 44 will pump the water stored in the hot and cold water chamber 30 to the hot and cold water channel 54 inside the hot and cold compress body 50 through the set of tube connectors 12 and the hot and cold water tube 541, and then circulate the water back into the hot and cold water chamber 30.

In the figures, the set of cold water tubes 531 and the hot and cold water tubes 541 are arranged parallel to each other and trapped in a thermal insulation protective cover, in order to avoid tangling the pipelines on one hand and to avoid the loss of temperature or energy during the transfer of cold water or hot water by the cold water tube 531 and the hot and cold water tube 541 on the other hand.

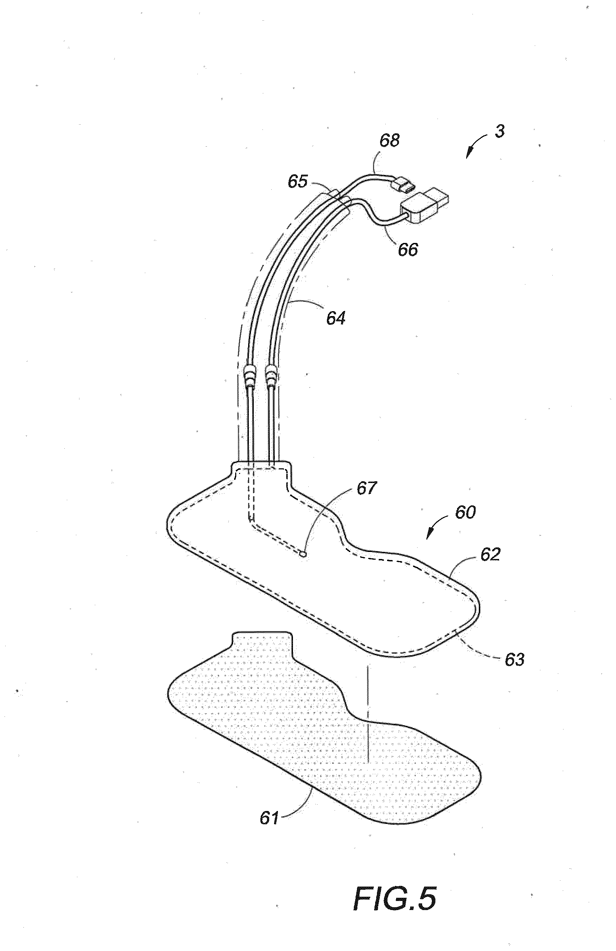

In FIGS. 2 and 5, the electric heating compress pad 3 includes a hot compress body 60 substantially in the shape of a sheet, the hot compress body 60 includes uniform temperature compress surface 61 flatly attachable to an affected part of a human body, a backside 62 opposite to the uniform temperature compress surface 61, an electric heating plate 63 installed between the uniform temperature compress surface 61 and the backside 62, and a flexible extension pad 64 extending from an edge of the hot compress body 60.

The tail end of the flexible extension pad 64 has a plug end 65 configured to be opposite to the plurality of tube connectors 12, a USB transmission cable 66 configured to be opposite to the USB port 13 and electrically connected to the electric heating plate 63. When the plug end 65 and the USB transmission cable 66 are plugably/unplugably inserted to the tube connector 12 and the USB port 13, the hot compress body 60 will be connected to the main system 1, and electrically connected to the electronic control system 40 of the main system 1 through the USB transmission cable 66 and the USB port 13.

In this way, the electronic control system 40 of the main system 1 can supply electric power to the electric heating plate 63 through the motherboard 42, USB port 13 and USB transmission cable 66, in order to heat the hot compress body 60. It is noteworthy that the hot compress body 60 of the electric heating compress pad 3 uses the electric heating plate 63 for heating and does not have any pipeline provided for circulating water, so that the plug end 65 plugged to the tube connector 12 is just used for fixing the electric heating compress pad 3 to the main system 1 to prevent the USB transmission cable 66 from falling off from the USB port 13.

In FIGS, 1, 2 and 6, the main system 1, hot and cold water compress pad 2 and electric heating compress pad 3 of the present disclosure are provided for executing the three functions of a quick cold compress, a hot and cold compress switch and a quick hot compress with high efficiency:

During a quick cold compress, the cold water tube 531 and the hot and cold water tube 541 of the hot and cold water compress pad 2 are connected to the tube connector 12 of the main system 1, and the hot and cold compress body 50 is attached to the affected part of a human body.

At this time, a user may set the operation interface 41, such that the motherboard 42 drives the refrigeration module 22 and the heating and refrigerating module 32 while refrigerating the water stored in the cold water chamber 20 and the hot and cold water chamber 30, and turns on the cold water pump 43, the hot and cold water pump 44 and the solenoid valve 45 to pump the cold water in the cold water chamber 20 and the hot and cold water chamber 30 to the hot and cold compress body 50 sequentially through the tube connector 12, the cold water tube 531 and the hot and cold water tube 541 for circulation, so that the cold water channel 53 and the hot and cold water channel 54 inside the hot and cold compress body 50 can circulate the cold water at the same time, and the uniform temperature compress surface 51 can conduct heat uniformly to achieve the quick cold compress effect.

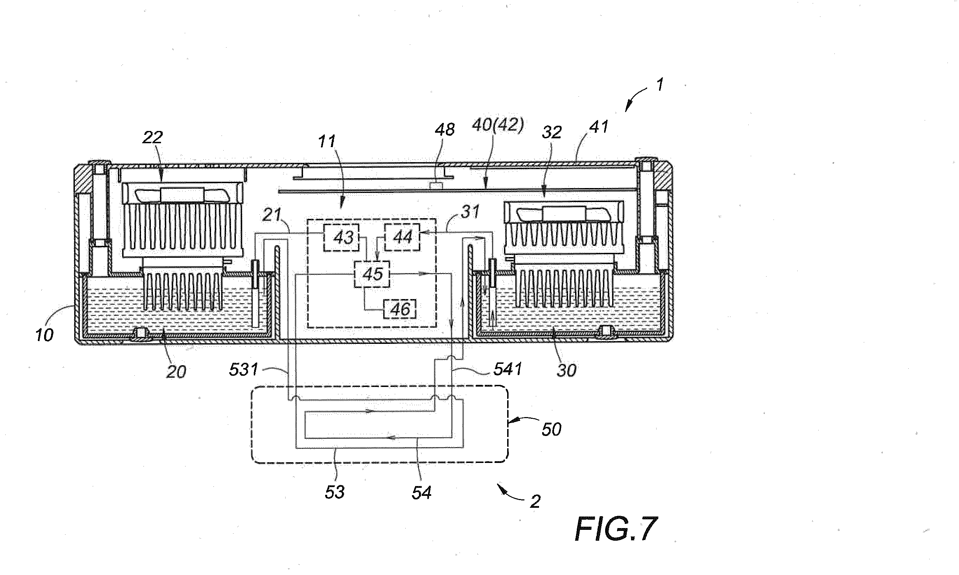

During a hot and cold compress switch as shown in FIGS. 1, 2 and 7, the cold water tube 531 and the hot and cold water tube 541 of the hot and cold water compress pad 2 are also connected to the tube connector 12 of the main system 1, and the hot and cold compress body 50 is attached to the affected part of a human body.

At this time, the user may set the operation interface 41 to let the motherboard 42 stop the operation of the refrigeration module 22 and the cold water pump 43, and drive the heating and refrigerating module 32 to heat the water stored in the hot and cold water chamber 30 and then turn off the hot and cold water pump 44 and solenoid valve 45 to let the hot water stored in the hot and cold water chamber 30 pass through the tube connector 12, the hot and cold water tube 541 to hot and cold water channel 54 the inside the hot and cold compress body 50, in order to increase the temperature of the hot and cold compress body 50 for the hot compress.

After a period of time of the hot compress, the motherboard 42 will stop the operation of the heating and refrigerating module 32 and the hot and cold water pump 44, while driving the refrigeration module 22 and the cold water pump 43 to start operating, so as to only allow the cold water stored in the hot and cold compress body 50 to circulate and thus accomplishing the hot and cold compress switch effect.

To enhance the hot and cold compress switch efficiency, the main system 1 of the present disclosure provides a function of emptying the cold water or hot water stored in the hot and cold compress body 50, thereby avoiding the cold water or hot water remained in the hot and cold compress body 50 from affecting the hot compress or cold compress effect, as well as providing a quick switch between cold compress and hot compress to improve the hot and cold compress efficiency.

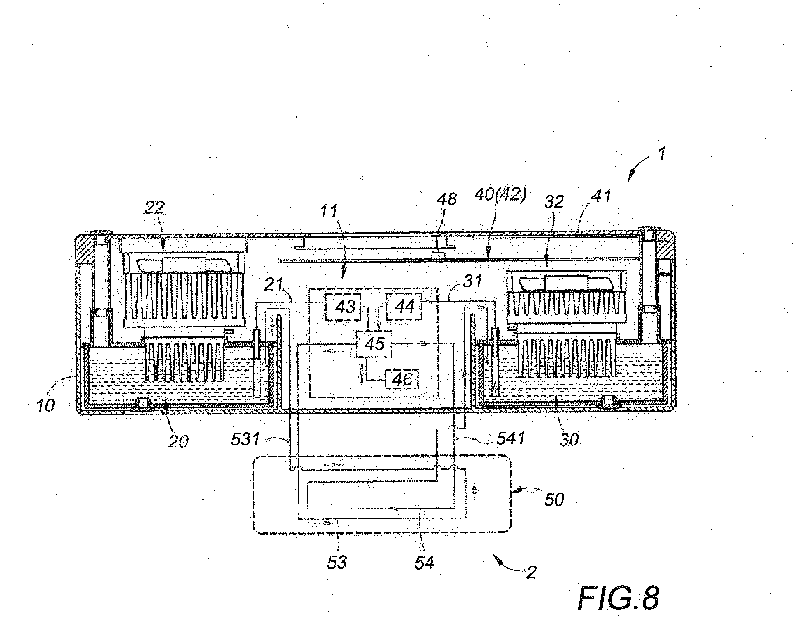

In a specific implementation method as shown in FIGS. 2 and 8, the electronic control system 40 further includes an air pump 46 electrically connected to the motherboard 42, disposed in the accommodation space 11, and respectively communicated to the cold water tube 531 and the hot and cold water tube 541 through the tube connector 12.

When a user wants to quickly switch from cold compress to hot compress, the user needs to stop the operation of the refrigeration module 22 and drive the heating and refrigerating module 32 to heat the water stored in the hot and cold water chamber 30. Alternatively, the user may drive the air pump 46 to pump air into the cold water tube 531 through the tube connector 12, so that.the cold water stored in the cold water channel 53 is pushed and emptied before returning to the cold water chamber 20, and only the hot water flows to the hot and cold water channel 54 inside the hot and cold compress body 50, so as to accomplish the function of quickly switching from cold compress to hot compress.

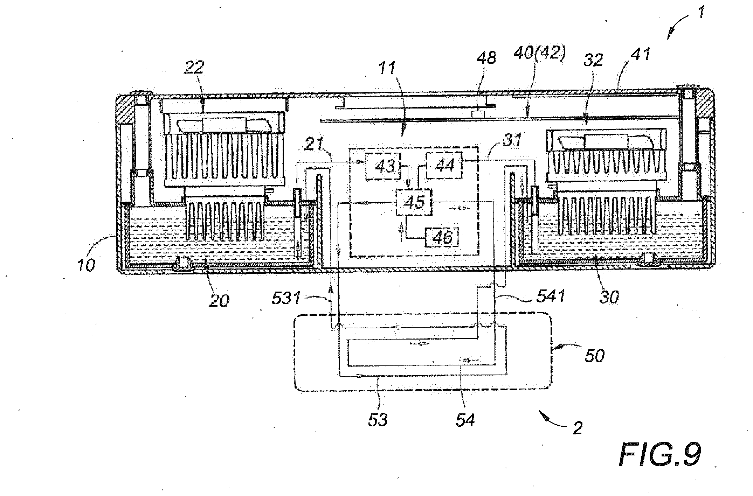

Similarly, when the user wants to switch from the hot compress to the cold compress as shown in FIG. 9, the motherboard 42 drives the air pump 46 to pump air into the hot and cold water tube 541, and to push, empty the hot water in the hot and cold water channel 54 of the hot and cold compress body 50 before returning the water into the hot and cold water chamber 30 and only allow the cold water to flow to the cold water channel 53 of the hot and cold compress body 50 to accomplish the function of switching the hot compress back to the cold compress.

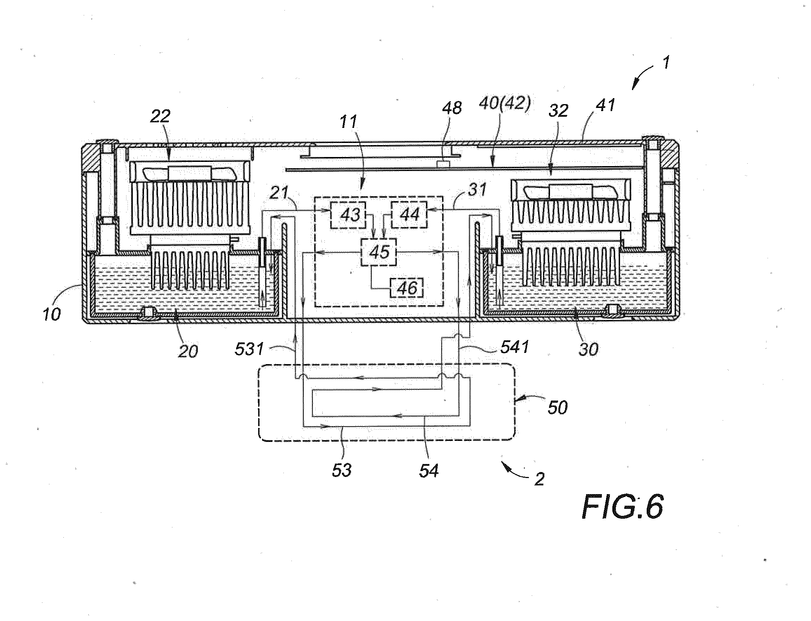

If it is necessary to improve the cold compress effect, the user may drive the heating and refrigerating module 32 to refrigerate the water stored in the hot and cold water chamber 30 and lower the temperature as shown in FIG. 6, and the hot and cold water pump 44 transfers the cold water to the hot and cold water channel 54 of the hot and cold compress body 50, so that cold water flows into the cold water channel 53 and the hot and cold water channel 54 inside the hot and cold compress body 50 at the same time to enhance the cold compress effect.

In order to increase the temperature difference between the cold compress and the hot compress to improve the hot and cold compress switch effect, a flexible electric heating plate 55 is installed between the uniform temperature compress surface 51 and the backside 52 of the hot and cold compress body 50 as shown in FIGS. 2, 4 and 10, and the electric heating plate 55 is in a shape corresponding to the shape of the gap between the cold water channel 53 and the hot and cold water channel 54 and arranged in a staggered fashion. In addition, a USB transmission cable 56 pluggable to the USB port 13 of the main system 1 is provided. When the hot water stored in the hot and cold water channel 54 is circulated, the electric heating plate 55 can obtain the power of the main system 1 through the USB transmission cable 56 to increase improve the efficiency of the hot and cold compress switch.

In conjunction with the implementation of the aforementioned air pump 46, when the electric heating plate 55 is used to assist in heating the hot and cold compress body 50, the air pump 46 can be driven to push and empty the cold water in the cold water channel 53 and then flow back to the cold water chamber 20, and only let the hot water flow through the hot and cold water channel 54 inside the hot and cold compress body 50 and heat the electric heating plate 55 to quickly switch the cold compress to hot compress.

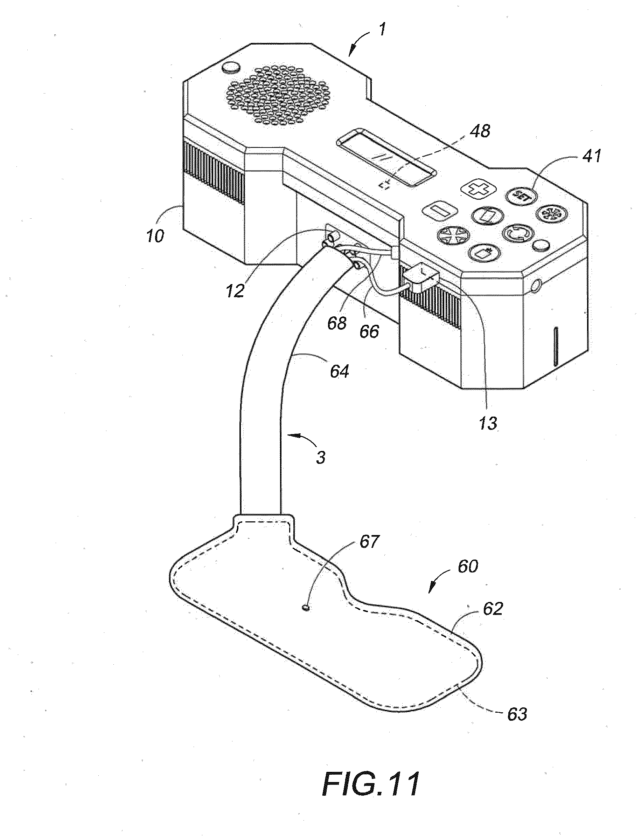

In FIGS. 1, 2, 4 and 11, when the present disclosure performs the quick hot compress, in addition to the use of the aforementioned hot and cold water compress pad 2, the hot and cold water compress pad 2 may be removed from the main system 1, and then the electric heating compress pad 3 is inserted onto the main system 1.

As described above, the hot and cold water compress pad 2 is plugably/unplugably connected to the tube connector 12 of the main system 1 by means of the cold water tube 531 and the hot and cold water tube 541, so that it only needs to remove the cold water tube 531 and the hot and cold water tube 541 from the tube connector 12 of the main system 1 and then the electric heating compress pad 3 uses the plug end 65 at the tail end of the flexible extension pad 64 to plug and connect the tube connector 12, so that after the electric heating compress pad 3 is connected to the main system 1, the USB transmission cable 66 is plugged into the USB port 13 of the main system 1 in order to obtain electric power by the main system 1. At this time, the uniform temperature compress surface 61 of the hot compress body 60 is flatly attached to the affected part of a human body, and the operation interface 41 and the motherboard 42 of the main system 1 controls the electric heating plate 63 inside the hot compress body 60 for heating in order to perform the quick hot compress.

In summation of the description above, the main system 1 of the present disclosure includes a cold water chamber 20 and a hot and cold water chamber 30, and a hot and cold water compress pad 2 and an electric heating compress pad 3 which can be interchangeably configured, wherein the hot and cold compress body 50 of the hot and cold water compress pad 2 further includes a cold water channel 53 and a hot and cold water channel 54 independent of each other, so that users can perform a quick cold compress, a hot and cold compress switch and a quick hot compress as needed to effectively overcome the drawbacks of the related-art hot and cold compress switch.

Other embodiments of the present disclosure are further described below:
In an embodiment as shown in FIGS. 2, 4 and 5, the casing 10 of the main system 1 further includes a signal interface 14 installed outside the accommodation space 11 and electrically connected to the motherboard 42, the hot and cold compress body 50 and the hot compress body 60 respectively include a temperature sensor 57, 67, the temperature sensor 57, 67 has a signal transmission cable 58, 68 plugably/unplugably connected to the signal interface 14 of the main system 1, so as to transfer the temperature of the hot and cold compress body 50 and the hot compress body 60 to the main system 1, which is provided for the electronic control system 40 to control the temperature.

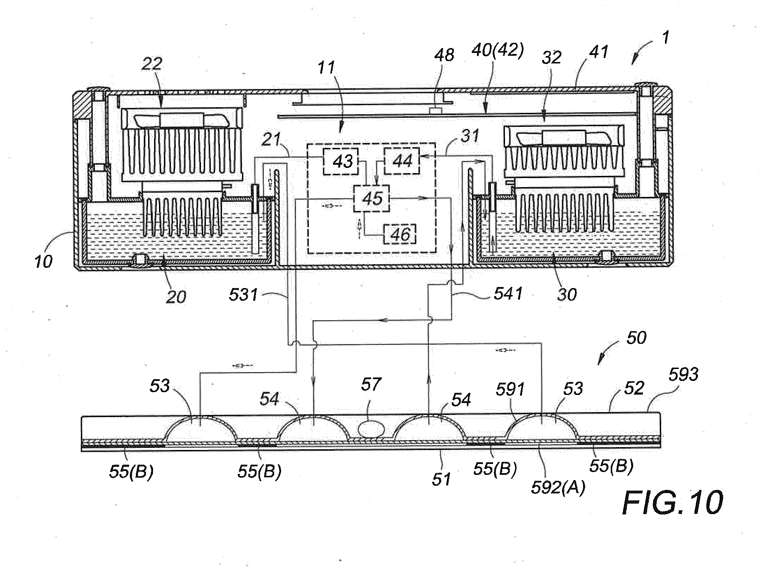

In an embodiment as shown in FIG. 10, the hot and cold compress body 50 is formed by superimposing and welding an upper flexible base material 591 and a lower flexible base material 592 with each other by a high temperature welding technology, the cold water channel 53, the hot and cold water channel 54 is disposed between the upper and lower flexible base materials 591, 592, the lower flexible base material 592 has a bottom A, the electric heating plate 55 is attached to the bottom A of the lower flexible base material 592, the uniform temperature compress surface 51 is attached to a bottom B of the electric heating plate 55, the uniform temperature compress surface 51 is formed by a composite film sheet containing graphene or a film sheet coated with graphene.

In an embodiment, the top of the upper flexible base material 591 is covered with a thermal insulation protective cover 593 to form the backside 52 of the hot and cold compress body 50, and the thermal insulation protective cover 593 can prevent the cold water or hot water circulated in the hot and cold compress body 50 from exchanging heat with the outside or losing energy.

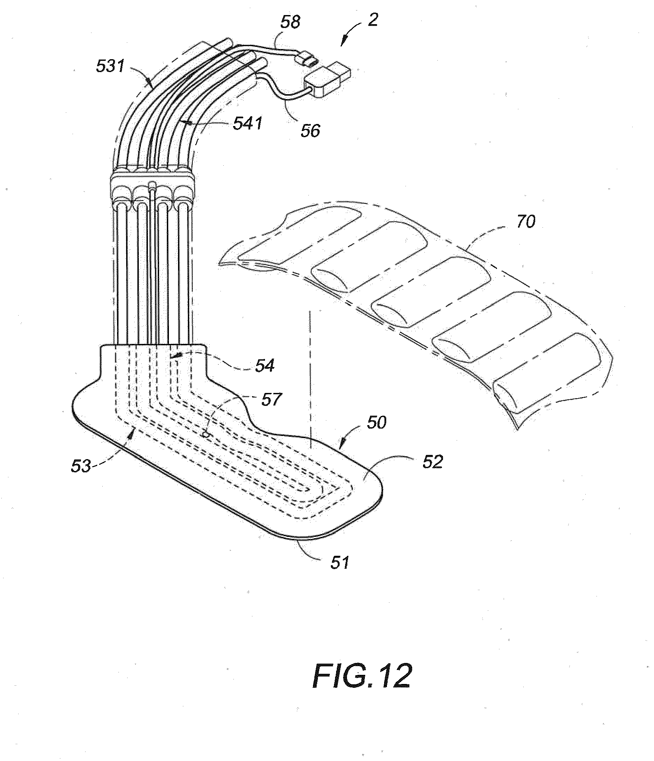

In an embodiment as shown in FIG. 1 and 12, the backsides 52, 62 of the hot and cold compress body 50 and the hot compress body 60 is provided with a fastener 70 respectively which is tied and fixed to an affected part of a human body (wherein this embodiment uses the hot and cold compress body 50 for example), the fastener 70 is a hook and loop tape, a buckle or a buckle with a plurality of pressurized air bags. When the fastener 70 is a pressurized air bag, the USB port 13 of the main system 1 can be used for supplying power to intermittently inflate and deflate the pressurized air bag, while the cold compress or hot compress achieves a massaging effect at the same time.

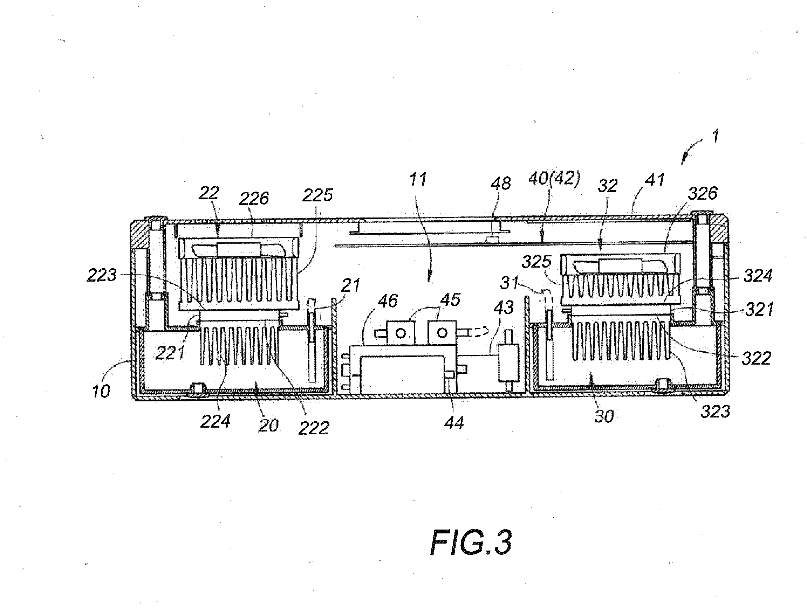

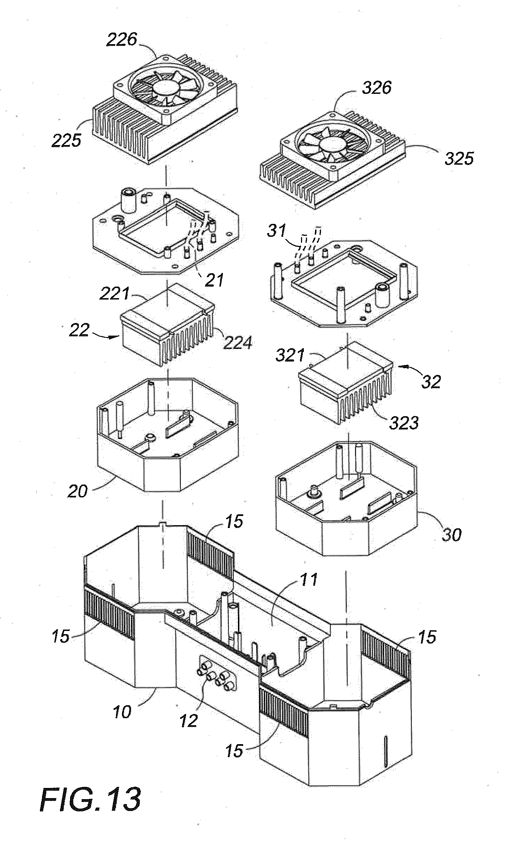

In an embodiment as shown in FIGS. 3 and 13, the refrigeration module 22 includes a refrigerating chip 221, the refrigerating chip 221 has a refrigerating surface 222 and a heat dissipation surface 223 opposite to the refrigerating surface 222, the refrigerating surface 222 is stacked with first temperature conduction fin 224 that extends into the cold water chamber 20, the heat dissipation surface 223 is stacked with a cooling fin 225, the top of the cooling fin 225 is provided with a fan 226 for blowing air and cooling the cooling fin 225, and the casing 10 has a plurality of heat dissipation holes 15 configured to be corresponsive to the surrounding and the top of the fan 226.

When the temperature of the refrigerating surface 222 of the refrigerating chip 221 drops, the first temperature conduction fin 224 can absorb the heat energy/temperature stored in the cold water chamber 20 to provide a refrigeration effect, and then the waste heat will be circulated and dissipated to the outside by means of the heat dissipation surface 223, the cooling fin 225, the fan 226 and the heat dissipation hole 15.

As described above, the heating and refrigerating module 32 is switched by reverse current or formed by a thermoelectric cooler (TEC), or a refrigerating chip and an electric heating plate (not shown in the figure). In the figures, if the heating and refrigerating module 32 is an electric heating and refrigerating element 321, its bottom 322 may be provided with a second temperature conduction fin 323 extending into the hot and cold water chamber 30, its top 324 may be provided with a cooling fin 325 and a fan 326, and the casing 10 has a plurality of heat dissipation holes 15 around the fan 326 to heat the hot and cold water stored in the hot and cold water chamber 30 through the second temperature conduction fin 323, The second temperature conduction fin 323 is used to heat or cool the water stored in the hot and cold water chamber 30, and the waste heat generated during the operation of the electric heating and refrigerating element 321 is dissipated through the cooling fin 325, the fan 326 and the heat dissipation hole 15.

In an embodiment as shown in FIGS. 1 and 2, the electronic control system 40 of the main system 1 further has a power input port 47 electrically connected to the motherboard 42, and a wireless transmission module 48, wherein the power input port 47 is provided for electrically connecting an external power supply (not shown in the figure), and power is outputted to the USB port 13 through the motherboard 42; the wireless transmission module 48 can transmit signals with an external mobile device 49, so that the external mobile device 49 can control the temperature of the hot and cold water compress pad 2 and the electric heating compress pad 3 at a remote end through the wireless transmission module 48 to facilitate the users' operation.

While the disclosure has been described by means of specific embodiments which are provided for illustrating the present disclosure only, but not intended for limiting the scope of the disclosure, and thus numerous modifications and variations with the same or similar objectives, structures, devices, and technical characteristics could be made thereto by those skilled in the art without departing from the scope and spirit of the disclosure as set forth in the claims.

## Claims

1. A hot and cold compress device with a dual water circuit design, comprising a main system (1) and at least two compress pads, the two compress pads being flexible and attachable to a surface of an affected part of human body, one of the two compress pads being a hot and cold water compress pad (2), and the other compress pad being an electric heating compress pad (3), **characterized in that**:
the main system (1) comprises a casing (10), which is separated into a cold water chamber (20) provided for storing cold water and installed with a refrigeration module (22), a hot and cold water chamber (30) for storing cold water or hot water and installed with a heating and refrigerating module (32), an accommodation space (11) formed between the cold water chamber (20) and the hot and cold water chamber (30) and provided with an electronic control system (40), and the casing (10) comprises a plurality of tube connectors (12) installed and configured relative to the exterior of the accommodation space (11), and at least one USB port (13) electrically coupled to the electronic control system (40);
the electronic control system (40) comprises an operation interface (41) installed outside the casing (10), and a motherboard (42), a cold water pump (43), a hot and cold water pump (44) and a set of solenoid valves (45) which are installed in the accommodation space (11) and electrically coupled to the operation interface (41), and the refrigeration module (22) and the heating and refrigerating module (32) are electrically coupled to the motherboard (42); wherein the cold water pump (43) is communicated to the set of solenoid valves (45) and cold water chamber (20) through a set of internal pipings (21), the hot and cold water pump (44) is communicated to the set of solenoid valves (45) and the hot and cold water chamber (30) through a set of internal pipings (31), such that the cold water pump (43) and the hot and cold water pump (44) can pump water in the cold water chamber (20) and the hot and cold water chamber (30) to the tube connector (12);
the hot and cold water compress pad (2) includes a hot and cold compress body (50) substantially in the shape of a sheet, the hot and cold compress body (50) includes a uniform temperature compress surface (51) attachable to an affected part of a human body, and a backside (52) opposite to the uniform temperature compress surface (51), a cold water channel (53) and a hot and cold water channel (54) separated from and independent of each other are disposed between the uniform temperature compress surface (51) and the backside (52), the cold water channel (53) and the hot and cold water channel (54) are arranged in a winding shape, and each of the cold water channel (53) and the hot and cold water channel (54) is plugably/unplugably inserted to the tube connectors (12) of the main system (1) through a set of cold water tubes (531) and a set of hot and cold water tubes (541) respectively;
the electric heating compress pad (3) includes a hot compress body (60) substantially in the shape of a sheet, the hot compress body (60) includes a uniform temperature compress surface (61) attachable to an affected part of a human body, a backside (62) opposite to the uniform temperature compress surface (61), an electric heating plate (63) installed between the uniform temperature compress surface (61) and the backside (62), and a flexible extension pad (64) installed at an edge of the electric heating compress pad (3); wherein the tail end of the flexible extension pad (64) is provided with a plug end (65) and a USB transmission cable (66) opposite to the tube connector (12) and the USB port (13) respectively and electrically coupled to the electric heating plate (63), such that the electric heating compress pad (3) is plugably/unplugably inserted and positioned to the tube connector (12) of the main system (1), and electrically coupled to the electronic control system (40) of the main system (1) after being inserted to the USB port (13) through the USB transmission cable (66);
during a quick cold compress, the cold water tube (531) and the hot and cold water tube (541) of the hot and cold water compress pad (2) are connected to the tube connector (12) of the main system (1), and the operation interface (41) is set, such that the motherboard (42) drives the refrigeration module (22) and the heating and refrigerating module (32) while cooling the water stored in the cold water chamber (20) and the hot and cold water chamber (30) simultaneously, and the cold water pump (43), the hot and cold water pump (44) and the solenoid valve (45) are turned on or off to pump the cold water in the cold water chamber (20) and the hot and cold water chamber (30) through the tube connector (12), the cold water tube (531) and the hot and cold water tube (541) into the hot and cold compress body (50) for circulation, and a quick cold compress is performed through the uniform temperature compress surface (51);
during a hot and cold compress switch, the cold water tube (531) and the hot and cold water tube (541) of the hot and cold compress body (50) are coupled to the tube connector (12) of the main system (1), and the operation interface (41) is set, such that the motherboard (42) can selectively drive the heating and refrigerating module (32) to heat the water stored in the hot and cold water chamber (30), and/or drive the refrigeration module (22) to cool the water stored in the cold water chamber (20), and the hot and cold water pump (44), the solenoid valve (45) and/or the cold water pump (43) are turned on or off to send the hot water and/or the cold water to the hot and cold water channel (54) and/or the cold water channel (53) of the hot and cold water compress pad (2) for circulation, such that the hot and cold compress body (50) can perform a hot and cold compress switch; and
during a quick hot compress, the hot and cold water compress pad (2) is removed from the main system (1), and the flexible extension pad (64) of the electric heating compress pad (3) is inserted to the tube connector (12) and the USB port (13) of the main system (1) through the plug end (65) and the USB transmission cable (66) respectively, and the operation interface (41) is set, such that the motherboard (42) can transmit electric power to the electric heating plate (63) through the USB port (13) and the USB transmission cable (66) for heating, to perform a quick hot compress at the uniform temperature compress surface (61) of the hot compress body (60).

2. The hot and cold compress device with a dual water circuit design according to claim 1, wherein the hot and cold compress body (50) further includes a flexible electric heating plate (55) installed between the uniform temperature compress surface (51) and the backside (52), the electric heating plate (55) is in a shape corresponding to that of the gap between the cold water channel (53) and the hot and cold water channel (54), arranged in a staggered fashion, and comprising a USB transmission cable (56) pluggable to the USB port (13) of the main system (1), and when hot water is circulated in the hot and cold water channel (54), the electric heating plate (55) obtains electric power through the USB transmission cable (56) for heating, so as to improve the efficiency of the hot and cold compress switch.

3. The hot and cold compress device with a dual water circuit design according to claim 1, wherein the electronic control system (40) further comprises an air pump (46) communicated to the cold water tube (531) and the hot and cold water tube (541) respectively, the air pump (46) is installed in the accommodation space (11) and electrically coupled to the motherboard (42); during a hot and cold compress switch, the motherboard (42) selectively drive the air pump (46) to inject air into the cold water tube (531) or the hot and cold water tube (541), so that the cold water or the hot water in the cold water channel (53) or the hot and cold water channel (54) of the hot and cold compress body (50) is pushed, emptied and returned to the corresponding cold water chamber (20) or hot and cold water chamber (30), so as to improve the efficiency of the hot and cold compress switch.

4. The hot and cold compress device with a dual water circuit design according to claim 1, wherein the casing (10) of the main system (1) further comprises a signal interface (14) disposed outside the accommodation space (11) and electrically coupled to the motherboard (42), the hot and cold compress body (50) and the hot compress body (60) comprise a temperature sensor (57), (67) respectively, the temperature sensor (57), (67) has a signal transmission cable (58), (68) pluggably and electrically coupled to the signal interface (14) of the main system (1), so as to transfer the temperature of the hot and cold compress body (50) and the hot compress body (60) to the main system (1).

5. The hot and cold compress device with a dual water circuit design according to claim 1, wherein the hot and cold compress body (50) is formed by superimposing and welding an upper flexible base material (591) and a lower flexible base material (592) with each other by a high-temperature welding technology, the cold water channel (53) and the hot and cold water channel (54) are disposed between the upper and lower flexible base materials (591), (592), the lower flexible base material (592) has a bottom (A), the electric heating plate (55) is attached to the bottom (A) of the lower flexible base material (592), the uniform temperature compress surface (51) is attached to a bottom (B) of the electric heating plate (55), the uniform temperature compress surface (51) is formed by a composite film sheet containing graphene or a film sheet coated with graphene; and the top of the upper flexible base material (591) is covered with a thermal insulation protective cover (593) to form the backside (52) of the hot and cold compress body (50).

6. The hot and cold compress device with a dual water circuit design according to claim 1, wherein the backsides (52), (62) of the hot and cold compress body (50) and the hot compress body (60) are respectively provided with a fastener (70) that can be tied and fixed to an affected part of a human body, the fastener (70) is a hook and loop tape, a fixed buckle or a fixed buckle with a plurality of pressurized air bags.

7. The hot and cold compress device with a dual water circuit design according to claim 1, wherein the refrigeration module (22) comprises a refrigerating chip (221) with a refrigerating surface (222) and a heat dissipation surface (223) opposite to the refrigerating surface (222), the refrigerating surface (222) is stacked with a first temperature conduction fin (224) that extends into the cold water chamber (20), the heat dissipation surface (223) is stacked with a cooling fin (225), the top of the cooling fin (225) is provided with a fan (226) capable of blowing air and cooling the cooling fin (225), and the surrounding and the top of the casing (10) corresponding to the fan (226) are provided with a plurality of heat dissipation holes (15).

8. The hot and cold compress device with a dual water circuit design according to claim 1, wherein the heating and refrigerating module (32) is switched to heating by reverse current or formed by a thermoelectric cooler (TEC), or a refrigerating chip capable of refrigerating the water in the hot and cold water chamber (30) and an electric heating plate capable of heating the water in the hot and cold water chamber (30).

9. The hot and cold compress device with a dual water circuit design according to claim 1, wherein the electronic control system (40) of the main system (1) further comprises a power input port (47), and a wireless transmission module (48) respectively and electrically coupled to the motherboard (42), and the power input port (47) is provided for electrically coupling an external power supply, and electric power is outputted to the USB port (13) through the motherboard (42); the wireless transmission module (48) is capable of transmitting signals to an external mobile device (49), and the external mobile device (49) is capable of controlling the temperature of the compress pad at a remote end through the wireless transmission module (48).
